# EUROPEAN PATENT SPECIFICATION

(11) **EP 0 831 926 B1**
(45) Date of publication and mention of the grant of the patent: **20.08.2003**
(21) Application number: 96916251.0
(22) Date of filing: 07.06.1996
(51) Int. Cl.: A61K 49/00

(54) **IMPROVEMENTS IN OR RELATING TO CONTRAST AGENTS**
VERBESSERUNGEN BEZÜGLICH KONTRASTMITTEL
AMELIORATION CONCERNANT DES AGENTS DE CONTRASTE

(30) Priority: 07.06.1995 GB 9511488
(43) Date of publication of application: 01.04.1998
(73) Proprietor: Amersham Health AS, 0401 Oslo (NO)
(72) Inventor: DUGSTAD, Harald, P.O. Box 4220 Torshov N-0401 Oslo (NO); KLAVENESS, Jo, P.O. Box 4220 Torshov N-0401 Oslo (NO); RONGVED, Pal, P.O. Box 4220 Torshov N-0401 Oslo (NO); SKURTVEIT, Roald, P.O. Box 4220 Torshov N-0401 Oslo (NO)
(74) Representative: Hammett, Audrey Grace Campbell
(86) International application number: GB9601362
(87) International publication number: WO96040275

(56) References cited:
- WO-A-92/17212
- WO-A-94/21302
- WO-A-94/28780
- WO-A-95/06518

## Description

This invention relates to aqueous dispersions of gas- or gas precursor-containing vesicles and to contrast agents of use in diagnostic imaging which may be obtained therefrom.

Published International Patent Application No. WO 92/17212 discloses ultrasound contrast agents comprising gas microbubbles or a gas precursor encapsulated by non-proteinaceous crosslinked or polymerised amphiphilic moieties. The surface active properties of the amphiphiles in such contrast agents stabilise the gas microbubbles, for example by reducing surface tension at their interfaces with surrounding liquid, e.g. a carrier liquid or body fluid, for example by forming monolayers or multilayers, e.g. one or more bilayers, at such interfaces, while chemical linking of the amphiphiles generates further stability.

The present invention concerns vesicle dispersions and contrast agents which fall within the overall disclosure of the above-mentioned WO 92/17212 but which are not specifically disclosed thereby, and is based on the finding that such vesicle dispersions and contrast agents in which the amphiphilic moieties comprise certain membrane-forming lipids may exhibit particularly advantageous properties.

As is recognised in the surfactant art, membrane-forming lipids may have the characteristic that they form liquid crystalline bilayers in aqueous media. Typically their molecular geometry is such that the hydrophilic and hydrophobic portions are of comparable size. Membrane-forming lipids also include amphiphiles which form monolayers or single bilayers at gas-water interfaces (e.g. as in Langmuir-Blodget films). Preferred membrane-forming lipids include lipids such as are found in biological membranes which are characterised by low water solubility and a tendency in aqueous solutions substantially to decrease surface tension, e.g. to almost zero; such lipids typically form gel state or liquid crystalline bilayers at low concentration in aqueous media.

We have now found that vesicles in which the amphiphilic moieties comprise one or more membrane-forming dialkanoylphosphatidylserine, dialkanoylphosphatidylglycerol, dialkanoylphosphatidylethanolamine and/or dialkanoylphosphatidylinositol lipids exhibit surprisingly high stability.' Contrast agents obtained by crosslinking or polymerising at least part of such membrane-forming lipid vesicles in the hydrophilic portion of the lipid(s) may exhibit a marked increase in contrast efficacy, for example as evidenced by enhanced grey-scale, Doppler and/or second harmonic efficacies, compared with the ultrasound contrast agents specifically disclosed in WO 92/17212.

Thus according to one aspect of the invention there is provided an aqueous dispersion of vesicles comprising gas microbubbles or a gas precursor stabilised by non-proteinaceous amphiphilic material comprising one or more membrane-forming lipids selected from dialkanoylphosphatidylserines, dialkanoylphosphatidylglycerols, dialkanoylphosphatidylethanolamines and dialkanoylphosphatidylinositols.

The invention also provides a diagnostic contrast agent comprising a vesicle dispersion as defined above which is injectable and wherein said membrane-forming lipid or lipids are at least partly crosslinked or polymerised in the hydrophilic portions thereof.

By virtue of the high degree of stability which may be imparted by the above-defined membrane-forming lipids it may be possible to incorporate other components, e.g. surfactants or cosurfactants, into the stabilising membrane-forming lipid material, even to the extent that the crosslinked or polymerised membrane-forming lipid in contrast agents of the invention represents a minor component of the stabilising, e.g. encapsulating, material, while maintaining adequate product stability. It may similarly be possible to employ a low degree of crosslinking or polymerisation in the contrast agents according to the invention, for example to enhance the flexibility of the encapsulating material, which in turn will enhance the image density afforded by the contrast agents.

The term "crosslinked" is used herein to denote chemical linking of at least two membrane-forming lipid molecules, e.g. to form a polymeric structure, and includes systems prepared by reaction with so-called zero crosslinking agents. The terms "polymerised" and "polymeric structure" include low molecular weight systems such as dimers and other oligomers. Oligomers, e.g. containing 2-20 repeating units, are one preferred category of membrane-forming lipids in accordance with the invention.

The membrane-forming lipids useful according to the invention are dialkanoylphosphatidylserines such as dipalmitoyl- and distearoyl-phosphatidylserines and corresponding dialkanoylphosphatidylglycerols, dialkanoylphosphatidylethanolamines and dialkanoylphosphatidylinositols.

The products of the invention may comprise a blend of membrane-forming lipids, e.g. such that the membrane-forming properties are superior to those of the individual components. Blends of membrane-forming lipids may, for example, include mixtures of dialkanoylphosphatidylserine and diacylphosphatidylcholine or of dialkanoylphosphatidylserine and diacylphosphatidic acid. Other components of such blends may include substances which modify membrane properties such as stability, dispersibility, aggregation tendency, biological activity, flexibility or polarity. Representative additives include sterols such as cholesterol, substances carrying surface-modifying groups such as polyethylene glycol moieties, and non-crosslinkable and non-polymerisable phospholipids. Alternatively the membrane-forming lipid may be subjected to a relatively low degree of crosslinking or polymerisation so that the encapsulating membrane material of the contrast agent product includes a proportion of unreacted (e.g. monomeric) membrane-forming lipid.

Any biocompatible gas may be employed in the vesicle dispersions and contrast agents of the invention, for example air, nitrogen, oxygen, hydrogen, nitrous oxide, carbon dioxide, helium, argon, sulphur fluorides such as sulphur hexafluoride, disulphur decafluoride and trifluoromethyl sulphur pentafluoride, low molecular weight (e.g. C₁₋₆) optionally fluorinated hydrocarbons such as methane, acetylene, carbon tetrafluoride and other perfluoroalkanes such as perfluoropropane, perfluorobutane and perfluoropentane, and mixture of any of the foregoing (e.g. as described in WO 95/03835). The term "gas" as used herein includes any substances, including mixtures, in gaseous or vapour form at 37°C. In general the gas may be free within the microbubbles or may be trapped or entrained within a containing substance.

Where the stabilising membrane has low permeability, e.g. as a result of having a high degree of crosslinking or polymerisation of the membrane-forming lipid, it may be preferred to employ a gas having relatively high solubility in water and body fluids. Where a more porous membrane, e.g. having a relatively low degree of crosslinking or polymerisation of membrane-forming lipid, is employed it may be preferred to employ a gas or gas mixture having low water solubility, for example comprising sulphur hexafluoride, disulphur decafluoride, a freon or a fluorocarbon such as perfluoroethane, perfluoropropane, perfluoropropylene, perfluorobutane, perfluorocyclobutane, perfluorobut-2-ene, perfluorobuta-1,3-diene, perfluorobut-2-yne or perfluoropentane. Other gases with low water solubility are disclosed in EP-A-0554213 and WO 93/05819.

Gas precursors include carbonates and bicarbonates, e.g. sodium or ammonium bicarbonate and aminomalonate esters. Other potential gas precursors are disclosed in WO 94/21302. The term "gas precursor" as used herein also embraces substances such as volatile hydrocarbons which may initially be encapsulated or otherwise stabilised but thereafter are partially or completely removed, e.g. by evaporation or freeze-drying, to be replaced by gas.

The contrast agents of the invention may be used in a variety of diagnostic imaging techniques, including ultrasound, MR and X-ray imaging; their use in diagnostic ultrasound imaging and in MR imaging, e.g. as susceptibility contrast agents, constitute preferred features of the invention.

For ultrasonic applications such as echocardiography, in order to permit free passage through the pulmonary system and to achieve resonance with the preferred imaging frequencies of about 0.1-15 MHz, it may be convenient to employ stabilised microbubbles having an average size of 0.1-10 µm, e.g. 1-7 µm. Substantially larger bubbles, e.g. with average sizes of up to 500 µm, may, however, be useful in other applications, for example gastrointestinal imaging or investigations of the uterus or Fallopian tubes.

The contrast agents according to the invention may be prepared by any convenient method, for example by forming an aqueous dispersion of vesicles comprising gas, a gas precursor or a volatile organic liquid stabilised by non-proteinaceous amphiphilic material comprising one or more membrane-forming lipids as defined above, crosslinking or polymerising at least part of said membrane-forming lipid or lipids in the hydrophilic portion thereof before, during or after such vesicle formation and, if necessary, removing the gas or liquid content of the vesicles and introducing a desired gas content.

It will be appreciated that in such a process any volatile organic liquid employed should desirably be at least substantially immiscible with water and possibly also a non-solvent for the membrane-forming lipid. Representative liquids may, for example, include halogenated hydrocarbons such as freons.

Vesicle formation may, for example, be effected by agitating an aqueous solution of the membrane-forming lipid, which may if desired initially be present in at least partially crosslinked or polymerised form, in the presence of an appropriate gas, gas precursor or organic liquid, for example by shaking or sonicating such a solution in a closed vessel also containing such gas and/or organic liquid so as to form vesicles having the desired size range. The aqueous solution is preferably prepared using degassed water and may, if desired, contain additional components such as a buffer, a viscosity enhancer etc. Where a gas is employed in this stage of the process it may advantageously be sulphur hexafluoride, a perfluoroalkane such as perfluorobutane or any other gas or gas mixture with similar low water solubility to ensure adequate persistence of the microbubbles during the time taken for formation of the stabilising membrane-forming lipid vesicles and their crosslinking or polymerisation.

Other vesicle formation techniques which may be employed include mechanical homogenisation, e.g. using a rotor-stator or colloid mill; microfluidisation, wherein jets of the two phase collide to form an emulsion; extrusion, e.g. wherein a two phase flow comprising large gas bubbles is passed through apertures such as a nozzle or the pores of a filter (which is preferably monosized) to generate an emulsion comprising smaller bubbles; and gas injection, e.g. wherein the gas is injected into liquid through apertures such as a nozzle or a microporous glass plate.

The membrane lipids may then, if necessary, be reacted with an appropriate crosslinking agent or polymerisation activating reagent which reacts with or promotes reaction of functional groups present in the hydrophilic portions of at least one membrane-forming lipid. It will be appreciated that reaction parameters such as reagent quantities, reaction time, temperature and pH, rate of stirring, presence and nature of any catalysts etc. may be adjusted as needed to obtain a desired degree of crosslinking or polymerisation. The degree of crosslinking may if desired be increased by use of e.g. trifunctional or other polyfunctional crosslinking agents.

The precise nature of the crosslinking agent or polymerisation activating reagent will clearly depend on the nature of the membrane-forming lipid, in particular on the nature of reactive functional groupings present therein. By way of example, the free amino groups of a dialkanoylphosphatidylserine or a dialkanoylphosphatidylethanolamine may be reacted with dialdehydes such as glutaraldehyde. The free amino and carboxyl groups of a dialkanoylphosphatidylserine may be polymerised to form amide polymers by reaction with a carboxyl group activator such as a carbodiimide, advantageously a water-soluble carbodiimide such as N-ethyl-N'-(3-dimethylaminopropyl)-carbodiimide hydrochloride - such compounds may also be viewed as zero crosslinking agents. It is also possible to condense two or more lipids respectively containing appropriate functional groups. Crosslinking agents which may be employed will generally contain at least two reactive functional groupings and include, for example, gem-dihalides, phosphoric acid and sulphonic acid.

It will be appreciated that the inherently high stability of the membrane-forming lipid-stabilised vesicles of the invention is advantageous in maintaining their vesicular structure during the course of such crosslinking and polymerisation reactions. The surprisingly high stability of the vesicles permits them to be washed or otherwise purified, and they may be stored for appreciable periods of time before being subjected to crosslinking or polymerisation. Processing times are therefore not critical and the process may if desired be interrupted.

After a desired degree of crosslinking or polymerisation has occurred residual reagent(s) may be quenched or removed, for example by removing the aqueous reaction mixture, e.g. using gravity separation or centrifugation. The crosslinked or polymerised vesicles may be washed as desired, e.g. 2-5 times; the wash water may if desired contain additives such as osmoregulators, buffers, cryoprotectants etc. Such washing steps may improve the size distribution characteristics of the product through removal of undersized microbubbles (which have minimal echogenic effect) and non-gas filled particles.

If it is desired to prepare a dry contrast agent product the washed vesicle dispersion may be dried and encapsulated gaseous or liquid core material removed, e.g. by lyophilisation, to give a dry powder comprising hollow thin-walled capsules which may be stored indefinitely, e.g. under an atmosphere of a gas or gas mixture which is desired to be incorporated into the product. The product may subsequently be reconstituted in, for example, sterile pyrogen-free water for injection to give an injectable contrast agent formulation.

The product may, if desired, be heat sterilised either before or after such a drying step.

The following non-limitative Examples serve to illustrate the invention.

### EXAMPLES 1-8

### GENERAL PROCEDURE:

1,2-Dipalmitoyl-sn-glycero-3-(phospho-L-serine) (sodium salt) was dissolved in a solution of 50 mg of propylene glycol/glycerol (3:10) in 1 ml water to a final concentration of 5 mg phospholipid/ml solution. 0.8 ml portions of this stock solution were transferred to 2 ml vials with screw caps, whereafter the head space was flushed with perfluorobutane gas. The vials were vigorously shaken for 45 seconds, and transferred to a table roller for approximately 30 minutes. Stock solutions of crosslinkers/polymerisation activating agents were prepared by dissolving the reagent in water to a concentration where 0.1 ml solution contained 1 equivalent of glutaraldehyde or 2 equivalents of N-ethyl-N'-(3-dimethylaminopropyl)carbodiimide hydrochloride (EDC). The reagent solution was added via a pipette tip to the bottom of each vial in a single application, whereafter the headspace was flushed with perfluorobutane gas, and the vial was transferred to the table roller and rolled for approximately 3 hours. The vials were centrifuged for 5 minutes at 2000 rpm at 20°C, whereafter the infranatant was removed from the bottom of the vial by a syringe and substituted with an equivalent volume of degassed water. The headspace was flushed with perfluorobutane gas, and rolling was continued until a homogeneous dispersion was obtained. This washing procedure was repeated twice.

### Example 1

The phospholipid was reacted with 0.1 ml glutaraldehyde solution. The product was characterised by Coulter counter analysis (number and size distribution) and in vitro echogenicity measurements, and was found to be stable at room temperature for several days.

### Example 2

The phospholipid was reacted with 0.1 ml EDC solution. The product was characterised by Coulter counter analysis (number and size distribution) and in vitro echogenicity measurements, and was found to be stable at room temperature for several days.

### Example 3

The phospholipid was prewashed for 30 minutes on the table roller, whereafter the vials were centrifuged for 5 minutes at 2000 rpm at 20°C, the infranatant was removed from the bottom of the vial by a syringe and substituted with an equivalent volume of degassed water. The headspace was flushed with perfluorobutane gas and rolling was continued until a homogeneous dispersion was obtained. This prewashed phospholipid was then reacted with 0.1 ml glutaraldehyde solution. The product was characterised by Coulter counter analysis (number and size distribution) and in vitro echogenicity measurements, and was found to be stable at room temperature for several days.

### Example 4

Phospholipid prewashed as in Example 3 was reacted with 0.1 ml EDC solution. The product was characterised by Coulter counter analysis (number and size distribution) and in vitro echogenicity measurements, and was found to be stable at room temperature for several days.

### Example 5

The phospholipid was reacted with 0.1 ml glutaraldehyde solution. The resulting suspension was frozen and lyophilised.

### Example 6

The phospholipid was reacted with 0.1 ml EDC solution. The resulting suspension was frozen and lyophilised.

### Example 7

The phospholipid was reacted with 0.1 ml glutaraldehyde solution. In the final washing step the infranatant was substituted with a 20% solution of glucose as cryoprotectant. The resulting suspension was frozen and lyophilised.

### Example 8

The phospholipid was reacted with 0.1 ml EDC solution. In the final washing step the infranatant was substituted with a 20% solution of glucose as cryoprotectant. The resulting suspension was frozen and lyophilised.

### Example 9

### a) Perfluoropentane/air microbubbles prepared using 1,2-dipalmitoyl-sn-glycero-3-(phospho-L-serine) (sodium salt)

A solution of 1,2-dipalmitoyl-sn-glycero-3-(phospho-L-serine) (sodium salt) (5.0 mg) in distilled water (1 ml) in a 2 ml vial with a septum was vigorously shaken for 15 seconds, heated to 60°C for 10 minutes and then cooled to 20°C. Perfluoropentane (1.2 µl was added and the vial was vigorously shaken for 30 seconds to give a suspension of perfluoropentane/air microbubbles of size 2-4 µm as determined by light microscopy. This suspension was stable for several days at room temperature.

### b) Polymerisation of the phospholipid

An aqueous solution of EDC (0.1 mg in 3 drops of water) was added to the aqueous suspension from (a) above. The vial was placed in a slowly rotating carousel, tilted by about 60°, for 20 hours at 20°C. The size of the resulting microbubbles, as estimated by light microscopy, was 2-4 µm. The suspension was stable for several days at room temperature.

### c) Crosslinking of the phospholipid

An aqueous solution of glutaraldehyde (25%, 16.5 mg) was added to the aqueous suspension from (a) above. The vial was placed in a slowly rotating carousel, tilted by about 60°, for 20 hours at 20°C. The size of the resulting microbubbles, as estimated by light microscopy, was 2-4 µm. The suspension was stable for several days at room temperature.

### Example 10

### Perfluoropentane/air microbubbles prepared using 1,2-dipalmitoyl-sn-glycero-3-(phospho-L-serine) (sodium salt)

A solution of 1,2-dipalmitoyl-sn-glycero-3-(phospho-L-serine) (sodium salt) (4.6 mg) in distilled water (1 ml) in a 2 ml vial with a septum was vigorously shaken for 15 seconds, heated to 60°C for 10 minutes and then cooled to 20°C. Perfluoropentane (2.4 µl) was added and the vial was vigorously shaken for 30 seconds to give a suspension of perfluoropentane/air microbubbles of size 2-5 µm as determined by light microscopy. This suspension was stable for several days at room temperature. The product may be polymerised or crosslinked using analogous techniques to those of Example 9(b) and (c).

### Example 11

### a) Perfluoropentane/air microbubbles prepared using 1,2-dipalmitoyl-sn-glycero-3-(phospho-L-serine) (sodium salt)

A solution of 1,2-dipalmitoyl-sn-glycero-3-(phospho-L-serine) (sodium salt) (5.2 mg) in distilled water (1 ml) in a 2 ml vial with a septum was vigorously shaken for 15 seconds, heated to 60°C for 10 minutes and then cooled to 20°C. Perfluoropentane (5 mg) was added and the vial was vigorously shaken for 30 seconds to give a suspension of perfluoropentane/air microbubbles of size 2-5 µm as determined by light microscopy. This suspension was stable for several days at room temperature.

### b) Crosslinking of the phospholipid

An aqueous solution of glutaraldehyde (25%, 16.5 mg) was added to the aqueous suspension from (a) above. The vial was placed in a slowly rotating carousel, tilted by about 60°, for 20 hours at 20°C. The size of the resulting microbubbles, as estimated by light microscopy, was 2-4 µm. The suspension was stable for several days at room temperature.

### Example 12

### a) Perfluorobutane/perfluorohexane microbubbles prepared using 1,2-dipalmitoyl-sn-glycero-3-(phospho-L-serine) (sodium salt)

A solution of 1,2-dipalmitoyl-sn-glycero-3-(phospho-L-serine) (sodium salt) (5.0 mg) in distilled water (1 ml) in a 2 ml vial with a septum was vigorously shaken for 15 seconds, heated to 60°C for 10 minutes and then cooled to 20°C. The vial was evacuated at 10 mm Hg for 20 minutes to remove air whereafter the headspace was flushed with perfluorobutane. Perfluorohexane (1.4 µl) was added and the vial was vigorously shaken for 30 seconds to give a suspension of perfluorobutane/perfluorohexane microbubbles of size 1-10 µm as determined by light microscopy. This suspension was stable for several days at room temperature.

### b) Polymerisation of the phospholipid

An aqueous solution of EDC (0.1 mg in 3 drops of water) was added to the aqueous suspension from (a) above. The vial was placed in a slowly rotating carousel, tilted by about 60°, for 20 hours at 20°C. The size of the resulting microbubbles, as estimated by light microscopy, was 2-4 µm. The suspension was stable for several days at room temperature.

### Example 13

### a) Perfluoropentane/air microbubbles prepared using 1,2-dipalmitoyl-sn-glycero-3-(phospho-L-serine) (sodium salt)

A solution of 1,2-dipalmitoyl-sn-glycero-3-(phospho-L-serine) (sodium salt) (3.0 mg) and propylene glycol/glycerol (3:10, 46 mg) in distilled water (1 ml) in a 2 ml vial with a septum was vigorously shaken for 1 minute. Perfluoropentane (4.5 µl) was added and the vial was vigorously shaken for 30 seconds to give a suspension of perfluoropentane/air microbubbles of size 2-10 µm as determined by light microscopy. This suspension was stable for several hours at room temperature.

### b) Polymerisation of the phospholipid

An aqueous solution of EDC (0.1 mg in 3 drops of water) was added to the aqueous suspension from (a) above and the resulting mixture was vigorously shaken for 5 seconds. The vial was then placed in a slowly rotating carousel, tilted by about 60°, for 20 hours at 20°C. The size of the resulting microbubbles, as estimated by light microscopy, was 1-8 µm. The suspension was stable for several days at room temperature.

### Example 14

### a) Perfluoropentane/air microbubbles prepared using 1,2-dipalmitoyl-sn-glycero-3-(phospho-L-serine) (sodium salt) and 1,2-dipalmitoyl-sn-glycero-3-phosphocholine

A solution of 1,2-dipalmitoyl-sn-glycero-3-(phospho-L-serine) (sodium salt) (2.5 mg) and 1,2-dipalmitoyl-sn-glycero-3-phosphocholine (0.5 mg) in distilled water (1 ml) was heated at 80°C for 1 hour. Perfluoropentane (1.2 µl) was then added and the mixture was vigorously shaken for 30 seconds to give a suspension of perfluoropentane/air microbubbles of size 1-8 µm as determined by light microscopy. This suspension was stable for several hours at room temperature.

### b) Crosslinking of the phospholipid

An aqueous solution of glutaraldehyde (25%, 16.5 mg) was added to the aqueous suspension from (a) above, and the mixture was vigorously shaken for 5 seconds. The vial was then placed in a slowly rotating carousel, tilted by about 60°, for 20 hours at 20°C. The size of the resulting microbubbles, as estimated by light microscopy, was 1-5 µm. The suspension was stable for several days at room temperature.

### Example 15

### a) Perfluoropentane/air microbubbles prepared using 1,2-dipalmitoyl-sn-glycero-3-(phospho-L-serine) (sodium salt) and 1,2-dipalmitoyl-sn-glycero-3-phosphocholine

A solution of 1,2-dipalmitoyl-sn-glycero-3-(phospho-L-serine) (sodium salt) (4.5 mg) and 1,2-dipalmitoyl-sn-glycero-3-phosphocholine (0.5 mg) in distilled water (1 ml) was heated at 80°C for 1 hour. Perfluoropentane (1.2 µl) was added and the mixture was vigorously shaken for 30 seconds to give a suspension of perfluoropentane/air microbubbles of size 1-10 µm as determined by light microscopy. This suspension was stable for several hours at room temperature.

### b) Crosslinking of the phospholipid

An aqueous solution of glutaraldehyde (25%, 16.5 mg) was added to the aqueous suspension from (a) above, and the mixture was vigorously shaken for 3 seconds. The vial was then placed in a slowly rotating carousel, tilted by about 60°, for 20 hours at 20°C. The size of the resulting microbubbles, as estimated by light microscopy, was 1-5 µm. The suspension was stable for several days at room temperature.

### c) Polymeristion of the phospholipid

An aqueous solution of EDC (0.1 mg in 3 drops of water) was added to the aqueous suspension from (a) above, and the mixture was vigorously shaken for 3 seconds The vial was then placed in a slowly rotating carousel, tilted by about 60°, for 20 hours at 20°C. The size of the resulting microbubbles, as estimated by light microscopy, was 1-5 µm. The suspension was stable for several days at room temperature.

### Example 16

### a) Perfluoropentane/air microbubbles prepared using 1,2-dipalmitoyl-sn-glycero-3-(phospho-L-serine) (sodium salt) and 1,2-dipalmitoyl-sn-glycero-3-phosphocholine

A solution of 1,2-dipalmitoyl-sn-glycero-3-(phospho-L-serine) (sodium salt) (2.5 mg) and 1,2-dipalmitoyl-sn-glycero-3-phosphocholine (0.7 mg) in distilled water (1 ml) was heated at 80°C for 1 hour. perfluoropentane (5 mg) was added and the mixture was vigorously shaken for 30 seconds to give a suspension of perfluoropentane/air microbubbles of size 1-8 µm as determined by light microscopy. This suspension was stable for several hours at room temperature.

### b) Polymerisation of the phospholipid

An aqueous solution of EDC (0.1 mg in 3 drops of water) was added to the aqueous suspension from (a) above, and the mixture was vigorously shaken for 3 seconds. The vial was then placed in a slowly rotating carousel, tilted by about 60°, for 20 hours at 20°C. The size of the resulting microbubbles, as estimated by light microscopy was 1-5 µm. The suspension was stable for several days at room temperature.

### Example 1

### a) Perfluoropentane/air microbubbles prepared using 1,2-dipalmitoyl-sn-glycero-3-(phospho-L-serine) (sodium salt) and 1,2-dipalmitoyl-sn-glycero-3-phosphocholine

A solution of 1,2-dipalmitoyl-sn-glycero-3-(phospho-L-serine) (sodium salt) (2.5 mg) and 1,2-dipalmitoyl-sn-glycero-3-phosphocholine (0.6 mg) and propylene glycol/glycerol (10, 40 mg) in distilled water (1 ml) was heated at 80°C for 1 hour. Perfluoropentane (1.4 µl) was added and one mixture was vigorously shaken for 30 seconds to give a suspension of perfluoropentane/air microbubbles of size 1-10 µm as determined by light microscopy. This suspension was stable for several hours at room temperature.

### b) Crosslinking of the phospholipid

An aqueous solution of glutaraldehyde (25%, 16.5 mg) was added to the aqueous suspension from (a) above, and the mixture was vigorously shaken for 3 seconds. The vial was then placed in a slowly rotating carousel, tilted by about 60°, for 20 hours at 20°C. The size of the resulting microbubbles, as estimated by light microscopy, was 1-5 µm. The suspension was stable for several days at room temperature.

### Example 18

### a) Perfluoropentane/air microbubbles prepared using 1,2-dipalmitoyl-sn-glycero-3-(phospho-L-serine) (sodium salt) and 1,2-dipalmitoyl-sn-glycero-3-phosphocholine

A solution of 1,2-dipalmitoyl-sn-glycero-3-(phospho-L-serine) (sodium salt) (4.5 mg) and 1,2-dipalmitoyl-sn-glycero-3-phosphocholine (1.0 mg) and propylene glycol/glycerol (3:10, 45 mg) in distilled water (1 ml) was heated at 80°C for 1 hour. Perfluoropentane (1.2 µl) was added and the mixture was vigorously shaken for 30 seconds to give a suspension of perfluoropentane/air microbubbles of size 1-10 µm as determined by light microscopy. This suspension was stable for several hours at room temperature.

### b) Crosslinking of the phospholipid

An aqueous solution of glutaraldehyde (25%, 16.5 mg) was added to the aqueous suspension from (a) above, and the mixture was vigorously shaken for 3 seconds. The vial was then placed in a slowly rotating carousel, tilted by about 60°, for 20 hours at 20°C. The size of the resulting microbubbles, as estimated by light microscopy, was 1-8 µm. The suspension was stable for several days at room temperature.

### c) Polymerisation of the phospholipid

An aqueous solution of EDC (0.1 mg in 3 drops of water) was added to the aqueous suspension from (a) above, and the mixture was vigorously shaken for 3 seconds. The vial was then placed in a slowly rotating carousel, tilted by about 60°, for 20 hours at 20°C. The size of the resulting microbubbles, as estimated by light microscopy, was 1-5 µm. The suspension was stable for several days at room temperature.

### Example 19

### a) Perfluoropentane/air microbubbles prepared using 1,2-dipalmitoyl-sn-glycero-3-(phospho-L-serine) (sodium salt) and 1,2-dipalmitoyl-sn-glycero-3-phosphocholine

A solution of 1,2-dipalmitoyl-sn-glycero-3-(phospho-L-serine) (sodium salt) (2.5 mg), 1,2-dipalmitoyl-sn-glycero-3-phosphocholine (0.5 mg) and propylene glycol/glycerol (3:10, 40 mg) in distilled water (1 ml) was heated at 80°C for 1 hour. Perfluoropentane (2.5 µl) was added and the mixture was vigorously shaken for 30 seconds to give a suspension of perfluoropentane/air microbubbles of size 1-10 µm as determined by light microscopy. This suspension was stable for several hours at room temperature.

### b) Crosslinking of the phospholipid

An aqueous solution of glutaraldehyde (25%, 16.5 mg) was added to the aqueous suspension from (a) above, and the mixture was vigorously shaken for 5 seconds. The vial was then placed in a slowly rotating carousel, tilted by about 60°, for 20 hours at 20°C. The size of the resulting microbubbles, as estimated by light microscopy, was 1-7 µm. The suspension was stable for several days at room temperature.

### c) Polymerisation of the phospholipid

An aqueous solution of EDC (0.1 mg in 3 drops of water) was added to the aqueous suspension from (a) above, and the mixture was vigorously shaken for 5 seconds. The vial was then placed in a slowly rotating carousel, tilted by about 60°, for 20 hours at 20°C. The size of the resulting microbubbles, as estimated by light microscopy, was 1-5 µm. The suspension was stable for several days at room temperature.

### Example 20

### a) Perfluorohexane/air microbubbles prepared using 1,2-dipalmitoyl-sn-glycero-3-(phospho-L-serine) (sodium salt) and 1,2-dipalmitoyl-sn-glycero-3-phosphocholine

A solution of 1,2-dipalmitoyl-sn-glycero-3-(phospho-L-serine) (sodium salt) (2.5 mg) and 1,2-dipalmitoyl-sn-glycero-3-phosphocholine (0.5 mg) in distilled water (1 ml) was heated at 80°C for 1 hour. Perfluorohexane (1.4 µl) was added and the mixture was vigorously shaken for 30 seconds to give a suspension of perfluorohexane/air microbubbles of size 2-10 µm as determined by light microscopy. This suspension was stable for several hours at room temperature.

### b) Polymerisation of the phospholipid

An aqueous solution of EDC (0.1 mg in 3 drops of water) was added to the aqueous suspension from (a) above, and the mixture was vigorously shaken for 3 seconds. The vial was then placed in a slowly rotating carousel, tilted by about 60°, for 20 hours at 20°C. The size of the resulting microbubbles, as estimated by light microscopy, was 2-10 µm. The suspension was stable for several days at room temperature.

### Example 21

### a) Perfluorohexane/air microbubbles prepared using 1,2-dipalmitoyl-sn-glycero-3-(phospho-L-serine) (sodium salt) and 1,2-dipalmitoyl-sn-glycero-3-phosphocholine

A solution of 1,2-dipalmitoyl-sn-glycero-3-(phospno-L-serine) (sodium salt) (2.5 mg) and 1,2-dipalmitoyl-sn-glycero-3-phosphocholine (1.3 mg) in distilled water (1 ml) was heated at 80°C for 1 hours. Perfluorohexane (1.4 µl) was added and the mixture was vigorously shaken for 30 seconds to give a suspension of perfluorohexane/air microbubbles of size 2-10 µm as determined by light microscopy. This suspension was stable for several hours at room temperature.

### b) Crosslinking of the phospholipid

An aqueous solution of glutaraldehyde (25%, 16.5 mg) was added to the aqueous suspension from (a) above, and the mixture was vigorously shaken for 3 seconds. The vial was then placed in a slowly rotating carousel, tilted by about 60°, for 20 hours at 20°C. The size of the resulting microbubbles, as estimated by light microscopy, was 2-10 µm. The suspension was stable for several days at room temperature.

### Example 22

### a) Perfluorohexane/air microbubbles prepared using 1,2-dipalmitoyl-sn-glycero-3-(phospho-L-serine) (sodium salt) and 1,2-dipalmitoyl-sn-glycero-3-phosphocholine

A solution of 1,2-dipalmitoyl-sn-glycero-3-(phospho-L-serine) (sodium salt) (2.5 mg) and 1,2-dipalmitoyl-sn-glycero-3-phosphocholine (0.6 mg) and propylene glycol/glycerol (3:10, 45 mg) in distilled water (1 ml) was heated at 80°C for 1 hour. Perfluorohexane (2.4 µl) was added and the mixture was vigorously shaken for 30 seconds to give a suspension of perfluorohexane/air microbubbles of size 2-10 µm as determined by light microscopy. This suspension was stable for several hours at room temperature.

### b) Crosslinking of the phospholipid

An aqueous solution of glutaraldehyde (25%, 16.5 mg) was added to the aqueous suspension from (a) above, and the mixture was vigorously shaken for 3 seconds. The vial was then placed in a slowly rotating carousel, tilted by about 60°, for 20 hours at 20°C. The size of the resulting microbubbles, as estimated by light microscopy, was 2-10 µm. The suspension was stable for several days at room temperature.

## Claims

1. An aqueous dispersion of vesicles comprising gas microbubbles or a gas precursor stabilised by non-proteinaceous amphiphilic material comprising one or more membrane-forming lipids selected from dialkanoylphosphatidylserines, dialkanoylphosphatidylglycerols, dialkanoylphosphatidylethanolamines and dialkanoylphosphatidylinositols.

2. A dispersion as claimed in claim 1 wherein said amphiphilic material comprises a blend of one or more dialkanoylphosphatidylserines with one or more diacylphosphatidylcholines and/or diacylphosphatidic acids.

3. A dispersion as claimed in claim 1 or claim 2 wherein said amphiphilic material further comprises a sterol.

4. A dispersion as claimed in any of the preceding claims wherein said amphiphilic material further comprises polyethylene glycol moieties.

5. A dispersion as claimed in any of the preceding claims comprising microbubbles of gas selected from air, nitrogen, oxygen, hydrogen, nitrous oxide, carbon dioxide, helium, argon, sulphur fluorides, optionally fluorinated low molecular weight hydrocarbons, and mixtures of any of the foregoing.

6. A dispersion as claimed in claim 5 comprising microbubbles of sulphur hexafluoride and/or one or more perfluorinated low molecular weight hydrocarbons.

7. A dispersion as claimed in claim 6 wherein said perfluorinated low molecular weight hydrocarbon is perfluoropropane, perfluorobutane or perfluoropentane.

8. A diagnostic contrast agent comprising a dispersion as claimed in any of the preceding claims which is injectable and wherein said membrane-forming lipid or lipids are at least partly crosslinked or polymerised in the hydrophilic portions thereof.

9. A contrast agent as claimed in claim 8 wherein said crosslinked or polymerised membrane-forming lipid or lipids are oligomers containing 2-20 repeating units.

10. Use of a contrast agent as claimed in claim 8 or claim 9 in the manufacture of an imaging agent for use in diagnostic imaging.

11. Use of a contrast agent as claimed in claim 8 or claim 9 in the manufacture of an imaging agent for use in diagnostic ultrasound imaging.

12. Use of a contrast agent as claimed in claim 8 or claim 9 in the manufacture of an imaging agent for use in magnetic resonance imaging.

13. A method of generating enhanced images of a human or non-human animal body which comprises generating an ultrasound or magnetic resonance image of at least a part of such a body which has been preadministered with a contrast agent as claimed in claim 8 or claim 9.

14. A process for the preparation of a contrast agent as claimed in claim 8 which comprises forming an aqueous dispersion of vesicles comprising gas, a gas precursor or a volatile organic liquid stabilised by non-proteinaceous amphiphilic material comprising one or more membrane-forming lipids selected from dialkanoylphosphatidylserines, dialkanoylphosphatidylglycerols, dialkanoylphosphatidylethanolamines and dialkanoylphosphatidylinositols, crosslinking or polymerising at least part of said membrane-forming lipid or lipids in the hydrophilic portion thereof before, during or after such vesicle formation and, if necessary, removing the gas or liquid content of the vesicles and introducing a desired gas content.

15. A process as claimed in claim 14 wherein an aqueous dispersion of vesicles comprising a volatile halogenated hydrocarbon, sulphur hexafluoride or a perfluoroalkane gas or gas mixture is formed.

16. A process as claimed in claim 14 or claim 15 wherein the dispersion of crosslinked or polymerised vesicles is subsequently lyophilised.

## Patentansprüche

1. Wässrige Disperson von Vesikeln, umfassend Gasmikrobläschen oder einen Gasvorläufer, stabilisiert durch nicht-proteinhaltiges, amphiphiles Material, umfassend ein oder meherere membranbildende Lipide, gewählt aus Dialkanoylphosphatidylserinen, Dialkanoylphosphatidylglycerinen, Dialkanoylphospatidylethanolaminen und Dialkanoylphosphatidylinositolen.

2. Dispersion nach Anspruch 1, wobei das amphiphile Material eine Mischung aus einem oder mehereren Dialkanoylphosphatidylserinen mit einem oder meheren Diacylphosphatidylcholinen und/oder Diacylphosphatidinsäuren umfasst.

3. Dispersion nach Anspruch 1 oder Anspruch 2, wobei das amphiphile Material weiterhin ein Sterol umfasst.

4. Dispersion nach mindestens einem der vorangehenden Ansprüche, wobei das amphiphile Material weiterhin Polyethylenglykol-Einheiten umfasst.

5. Dispersion nach mindestens einem der vorangehenden Ansprüche, umfassen Mikrobläschen aus Gas, gewählt aus Luft, Stickstoff, Sauerstoff, Wasserstoff, Stickstoff(I)-oxid, Kohlendioxid, Helium, Argon, Schwefelfluoriden, wahlweise fluorierten niedermolekulargewichtigen Kohlenwasserstoffen und Mischungen aus beliebigen der Vorangehenden.

6. Dispersion nach Anspruch 5, umfassend Mikrobläschen aus Schwefelhexafluorid und/oder ein oder mehrere perfluorierte niedermolekulargewichtige Kohlenwasserstoffe.

7. Dispersion nach Ansrpruch 6, wobei der perfluorierte niedermolekulargewichtige Kohlenwasserstoff Perfluorpropan, Perfluorbutan oder Perfluorpentan ist.

8. Diagnostisches Kontrastmittel, umfassend eine Dispersion nach mindestens einem der vorangehenden Ansprüche, welche injizierbar ist, und wobei das membranbildende Lipid oder die Lipide zumindest teilweise in den hydrophilen Bereichen hiervon vernetzt oder polymerisiert sind.

9. Kontrastmittel nach Anspruch 8, wobei das vernetzte oder polymerisierte, membranbildende Lipid oder Lipide Oligomere sind, welche 2-20 Wiederholungseinheiten enthalten.

10. Verwendung eines Kontrastmittels nach Anspruch 8 oder Anspruch 9 bei der Herstellung eines Bilderzeugungsmittels zur Verwendung in der diagnostischen Bilderzeugung.

11. Verwendung eines Kontrastmittels nach Anspruch 8 oder Anspruch 9 bei der Herstellung eines Bildherstellungsmittels zur Verwendung einer diagnostischen Ultraschall-Bilderzeugung.

12. Verwendung eines Kontrastmittels nach Anspruch 8 oder Anspruch 9 bei der Herstellung eines Bilderzeugungsmittels zur Verwendung bei der Magnetresonanz-Bilderzeugung.

13. Verfahren zur Erzeugung verstärkter Bilder eines menschlichen oder nicht menschlichen tierischen Körpers, umfassend das Erzeugen eines Ultraschalloder Magnetresonanz-Bildes mindestens eines Teils eines solchen Körpers, welchem vorausgehend ein Kontrastmittel nach Anspruch 8 oder Anspruch 9 verabreicht worden ist.

14. Verfahren zur Herstellung eines Kontrastmittels nach Anspruch 8, umfassend das Bilden einer wässrigen Dispersion von Vesikeln, umfassend Gas, einen Gasvorläufer oder eine flüchtige organische Flüssigkeit, stabilisiert durch nicht-proteinhaltiges amphiphiles Material, umfassend ein oder mehrere membranbildende Lipide, gewählt aus Dialkanoylphasphatidylserinen, Dialkanoylphosphatidylglycerinen, Dialkanoylphosphatidylethanolaminen und Dialkanoylphosphatidylinositolen, Vernetzen oder Polymerisieren mindestens eines Teils des membranbildenden Lipids oder der Lipide in dem hydrophilen Bereich hiervon, vor, während oder nach einer solchen Vesikelbildung und, falls notwendig, Entfernen des Gases oder des Flüssigkeitsgehaltes der Vesikel und Einführen eines erwünschten Gasgehaltes.

15. Verfahren nach Anspruch 14, wobei eine wässrige Dispersion von Vesikeln, umfassend einen flüchtigen halogenierten Kohlenwasserstoff, Schwefelhexafluorid oder ein Perfluoralkangas oder Gasmischung, gebildet wird.

16. Verfahren nach Anspruch 14 oder Anspruch 15, wobei die Dispersion aus vernetzten oder polymerisierten Vesikeln nachfolgend lyophilisiert wird.

## Revendications

1. Dispersion aqueuse de vésicules comprenant des microbulles de gaz ou un précurseur de gaz stabilisées par un matériau amphiphile non protéique comprenant un ou plusieurs lipides formant une membrane choisis parmi les dialcanoylphosphatidylsérines, les dialcanoylphosphatidylglycérols, les dialcanoylphosphatidyléthanolamines et les dialcanoylphosphatidylinositols.

2. Dispersion selon la revendication 1, dans laquelle ledit matériau amphiphile comprend un mélange d'une ou plusieurs dialcanoylphosphatidylsérines avec une ou plusieurs diacylphosphatidylcholines et/ou acides diacylphosphatidiques.

3. Dispersion selon la revendication 1 ou la revendication 2, dans laquelle ledit matériau amphiphile comprend en outre un stérol.

4. Dispersion selon l'une quelconque des revendications précédentes, dans laquelle ledit matériau amphiphile comprend en outre des fractions de polyéthylèneglycol.

5. Dispersion selon l'une quelconque des revendications précédentes, comprenant des microbulles de gaz choisi parmi l'air, l'azote, l'oxygène, l'hydrogène, l'oxyde nitreux, le dioxyde de carbone, l'hélium, l'argon, les fluorures de soufre, les hydrocarbures de bas poids moléculaire éventuellement fluorés, et leurs mélanges.

6. Dispersion selon la revendication 5, comprenant des microbulles d'hexafluorure de soufre et/ou d'un ou plusieurs hydrocarbures de bas poids moléculaire perfluorés.

7. Dispersion selon la revendication 6, dans laquelle ledit hydrocarbure de bas poids moléculaire perfluoré est le perfluoropropane, le perfluorobutane ou le perfluoropentane.

8. Agent de contraste diagnostique comprenant une dispersion selon l'une quelconque des revendications précédentes qui est injectable et dans lequel ledit ou lesdits lipides formant une membrane sont au moins partiellement réticulés ou polymérisés dans leurs parties hydrophiles.

9. Agent de contraste selon la revendication 8, dans lequel ledit ou lesdits lipides formant une membrane réticulés ou polymérisés sont des oligomères contenant 2 à 20 motifs répétés.

10. Utilisation d'un agent de contraste selon la revendication 8 ou la revendication 9 dans la fabrication d'un agent d'imagerie pour l'utilisation en imagerie diagnostique.

11. Utilisation d'un agent de contraste selon la revendication 8 ou la revendication 9 dans la fabrication d'un agent d'imagerie pour l'utilisation en imagerie diagnostique par ultrasons.

12. Utilisation d'un agent de contraste selon la revendication 8 ou la revendication 9 dans la fabrication d'un agent d'imagerie pour l'utilisation en imagerie par résonnance magnétique.

13. Procédé pour générer des images rehaussées d'un corps animal humain ou non humain, qui comprend le fait de générer une image par ultrasons ou résonnance magnétique d'au moins une partie d'un tel corps qui a reçu une préadministration d'un agent de contraste tel que revendiqué dans la revendication 8 ou la revendication 9.

14. Procédé pour la préparation d'un agent de contraste tel que revendiqué dans la revendication 8, qui comprend le fait de former une dispersion aqueuse de vésicules comprenant un gaz, un précurseur de gaz ou un liquide organique volatil stabilisées par un matériau amphiphile non protéique comprenant un ou plusieurs lipides formant une membrane choisis parmi les dialcanoylphosphatidylsérines, les dialcanoylphosphatidylglycérols, les dialcanoylphosphatidyléthanolamines et les dialcanoylphosphatidylinositols, de réticuler ou polymériser au moins une partie dudit ou desdits lipides formant une membrane dans leur partie hydrophile, avant, pendant ou après la formation de telles vésicules et, si nécessaire, d'éliminer le contenu gazeux ou liquide des vésicules et d'introduire un contenu gazeux souhaité.

15. Procédé selon la revendication 14, dans lequel une dispersion aqueuse de vésicules comprenant un gaz d'hydrocarbure halogéné volatil, d'hexafluorure de soufre ou de perfluoroalcane ou un mélange gazeux est formée.

16. Procédé selon la revendication 14 ou la revendication 15, dans lequel la dispersion de vésicules réticulées ou polymérisées est ensuite lyophilisée.
